(19) Europäisches Patentamt
European Patent Office
Office européen des brevets

(11) Veröffentlichungsnummer: **0 033 516**
**A1**

(12) # EUROPÄISCHE PATENTANMELDUNG

(21) Anmeldenummer: 81100630.3

(22) Anmeldetag: 29.01.81

(51) Int. Cl.³: C 07 D 307/32
A 01 N 43/08

(30) Priorität: 31.01.80 CH 782/80
21.11.80 CH 8623/80

(43) Veröffentlichungstag der Anmeldung: 12.08.81 Patentblatt 81/32

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Dorn, Franz, Dr.
Bohnackerstrasse 5
CH-8157 Dielsdorf(CH)

(72) Erfinder: Zobrist, Peter, Dr.
Riedterstrasse 23
CH-8162 Steinmaur(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Substituierte Anilide, Herstellung dieser Verbindungen, fungizide Mittel, die diese Verbindungen als Wirkstoffe enthalten sowie Verwendung solcher Verbindungen und Mittel zur Bekämpfung von Fungi in der Landwirtschaft und im Gartenbau.**

(57) Die Erfindung betrifft substituierte Anilide der allgemeinen Formel

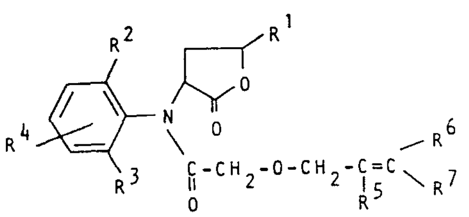

1

worin $R^1$ Wasserstoff oder Methyl, $R^2$ und $R^3$ $C_{1-3}$-Alkyl, $C_{1-3}$-Alkoxy oder Halogen, $R^4$ Wasserstoff, $C_{1-3}$-Alkyl oder Halogen und $R^5$,$R^6$ und $R^7$ Wasserstoff oder Methyl bedeuten, wobei $R^5$ zusammen mit $R^6$ auch eine zusätzliche C-C-Bindung darstellen können und wobei die Gesamtzahl der Kohlenstoffatome in den Substituenten $R^2$, $R^3$ und $R^4$ 6 nicht übersteigt,
ein Verfahren zu deren Herstellung, fungicide Mittel, die diese Verbindungen als Wirkstoffe enthalten sowie die Verwendung solcher Verbindungen bzw. Mittel zur Bekämpfung von Fungi in der Landwirtschaft und im Gartenbau.

EP 0 033 516 A1

2 9. Jan. 1981
0033516

F.Hoffmann-La Roche & Co.Aktiengesellschaft, Basel/Schweiz

RAN 6103/13

Substituierte Anilide, Herstellung dieser Verbindungen, fungizide Mittel, die diese Verbindungen als Wirkstoffe enthalten sowie Verwendung solcher Verbindungen und Mittel zur Bekämpfung von Fungi in der Landwirtschaft und im Gartenbau.

Die Erfindung betrifft Verbindungen der allgemeinen Formel

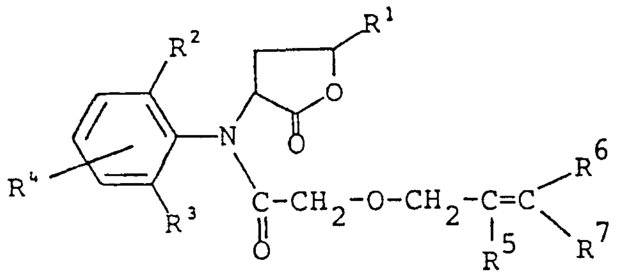

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt.

Die Verbindungen der Formel I besitzen fungicide Eigenschaften und eignen sich als fungicide Wirkstoffe.

Pa/ 27.10.1980

Die Erfindung betrifft ferner ein Verfahren zur Herstellung der Verbindungen der Formel I, Verbindungen der Formel I als fungicide Wirkstoffe, fungicide Mittel, die Verbindungen der Formel I als Wirkstoffe enthalten sowie die Verwendung solcher Verbindungen und fungicider Mittel zur Bekämpfung von Pilzen in der Landwirtschaft und im Gartenbau.

Unter Alkyl und dem Alkylteil einer Alkoxygruppe in der Formel I sind je nach Anzahl der angegebenen Kohlenstoffatome folgende Gruppen zu verstehen: Methyl, Aethyl, n-Propyl und Isopropyl.

Der Ausdruck "Halogen" umfasst Fluor, Chlor, Brom und Jod, wobei Chlor bevorzugt ist.

$R^1$ bedeutet vorzugsweise Wasserstoff.

Hat $R^2$ oder $R^3$ die Bedeutung Alkyl mit 1 bis 3 Kohlenstoffatomen, so ist dies vorzugsweise Methyl.

$R^4$ bedeutet vorzugsweise Wasserstoff, so dass die substituierte Phenylgruppe der Verbindungen der Formel I vorzugsweise 2,6-disubstituiert ist. Insbesondere steht diese Gruppe für 2,6-Dichlorphenyl, 2-Chlor-6-methylphenyl oder 2,6-Dimethylphenyl.

Besonders bevorzugte Verbindungen der Formel I sind das 2-Allyloxy-N-(tetrahydro-2-oxo-3-furyl)-2',6'-acetoxylidid und das 2-(2-Propinyloxy)-N-(tetrahydro-2-oxo-3-furyl)-2',6'-acetoxylidid.

In den Verbindungen der Formel I sind asymmetrische Kohlenstoffatome vorhanden, so dass die Verbindungen als optische Antipoden vorliegen können. Unabhängig davon kommt auch in gewissen Fällen eine Atropisomerie vor. Durch das Vorliegen einer Doppelbindung in den Verbindungen der Formel I kann bei diesen Verbindungen zusätzlich geometrische

Isomerie auftreten. Die Formel I soll demnach all diese möglichen isomeren Formen umfassen.

Das erfindungsgemässe Verfahren zur Herstellung der Verbindungen der Formel I ist dadurch gekennzeichnet, dass man eine Verbindung der Formel

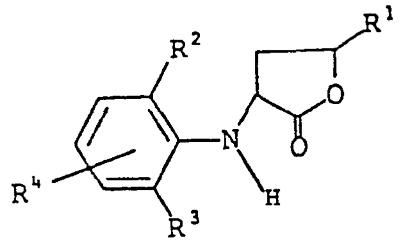

II

worin $R^1$, $R^2$, $R^3$ und $R^4$ die oben angegebenen Bedeutungen besitzen,

mit einer Carbonsäure der Formel

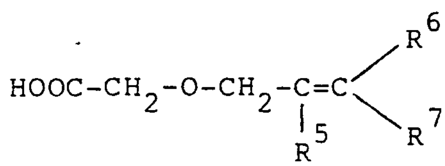

III

worin $R^5$, $R^6$ und $R^7$ die oben angegebenen Bedeutungen besitzen,

oder einem reaktionsfähigen Säurehalogenid, Säureanhydrid, Ester oder Amid, vorzugsweise mit einem Säurehalogenid oder dem Säureanhydrid, davon, umsetzt.

Die Umsetzung kann in An- oder Abwesenheit von gegenüber den Reaktionsteilnehmern inerten Lösungs- oder Verdünnungsmitteln durchgeführt werden, z.B. aliphatischen und aromatischen Kohlenwasserstoffen wie Benzol, Toluol, Xylolen und Petroläthern; halogenierten Kohlenwasserstoffen wie Chlorbenzolen, Methylenchlorid, Aethylenchlorid und Chloroform; Aethern und ätherartigen Verbindungen wie Dialkyläthern, Dioxan und Tetrahydrofuran; Nitrilen wie Acetonitril; N,N-dialkylierten Amiden wie Dimethylformamid; Dimethylsulfoxid; Ketonen wie Methyläthylketon; und Gemischen

solcher Lösungsmittel untereinander.

Die Reaktionstemperaturen liegen zweckmässigerweise zwischen 0 und 180°C, vorzugsweise zwischen 20 und 120°C. In manchen Fällen ist die Verwendung von säurebindenden Mitteln bzw. Kondensationsmitteln vorteilhaft. Als solche kommen tertiäre Amine wie Trialkylamine (z.B. Triäthylamin), Pyridin und Alkylpyridine, oder anorganische Basen, wie die Oxide und Hydroxide, Hydrogencarbonate und Carbonate von Alkali- und Erdalkalimetallen sowie Natriumacetat in Betracht. Als säurebindendes Mittel kann ausserdem ein Ueberschuss des jeweiligen Anilinderivates der Formel II dienen.

Als Säurehalogenide der Verbindungen der Formel III sind die Säurechloride oder Säurebromide bevorzugt.

Die Isolierung und Reinigung der so hergestellten Verbindungen der Formel I erfolgen nach an sich bekannten Methoden.

Sofern keine gezielte Synthese zur Isolierung reiner Isomerer durchgeführt wird, fällt normalerweise ein Produkt als Gemisch zweier oder mehrerer Isomerer an. Die Isomeren können nach bekannten Methoden aufgetrennt werden.

Die als Ausgangsmaterialien dienenden Verbindungen der Formel II und III sowie reaktionsfähigen Säurehalogenide, Säureanhydride, Ester und Amide der Verbindungen der Formel III sind entweder bekannt oder können nach an sich bekannten Methoden hergestellt werden.

Die Verbindungen der Formel I zeigen auf Pflanzen, die von Pilzen befallen sind, eine spezifische fungicide Wirkung. Als Pflanzen können im Rahmen der vorliegenden Erfindung beispielsweise Mais, Gemüse, Zuckerrüben, Soja, Obstbäume, Zierpflanzen, vor allem aber Reben, Hopfen, Gurkengewächse (Gurken, Kürbis, Melonen), Solanaceen wie Kartoffeln, Tabak und Tomaten, sowie auch Bananen-, Ananas-, Avocado-, Kakao- und Naturkautschuk-Gewächse genannt werden.

Die Verbindungen der Formel I sind ferner wirksam gegen Eier von beispielsweise Spinnentieren und eignen sich somit zur Einarbeitung als Wirkstoffe auch in ovicide Mittel.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe eignen sich insbesondere zur Bekämpfung von Pilzen der Gattungen Phytophthora, Peronospora, Pseudoperonospora, Pythium, Bremia und Plasmopara. Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Verbindungen der Formel I wirken unter Gewächshausbedingungen bereits bei einer Konzentration von 5 mg bis 500 mg Wirksubstanz pro Liter Spritzbrühe. Im Freiland werden vorteilhaft Konzentrationen von 100 g bis 2500 g Wirksubstanz der Formel I pro Hektar und Behandlung zur Anwendung gebracht. Beispielsweise wird zur erfolgreichen Rebmehltaubekämpfung zweckmässigerweise eine Konzentration von 200 g bis 1000 g, vorzugsweise 200 g bis 600 g, Wirksubstanz pro Hektar und Behandlung benützt.

Ein Teil der Verbindungen der Formel I zeichnet sich durch hohe systemische Wirkungsentfaltung aus.

Durch Sekundärverteilung der Wirksubstanz (Gasphasenwirkung) können zusätzlich auch nicht behandelte Pflanzenteile geschützt werden.

Für praktische Zwecke können die Verbindungen der Formel I als für Wirbeltiere weitgehend ungiftig qualifiziert werden.

Die Verbindungen der Formel I können in verschiedenartigen Mitteln, z.B. Spritzbrühen, wässrigen Suspensionen, Emulsionen, emulgierbaren Konzentrationen und pulverförmigen Präparaten, formuliert werden. Die erfindungsgemässen fungiciden Mittel sind dadurch gekennzeichnet, dass sie eine wirksame Menge von mindestens einer Verbindung der allgemeinen Formel I, wie oben definiert, sowie inertes Trägermaterial enthalten.

Im allgemeinen enthält ein Mittel, je nach dessen Art, zwischen 0,0001 und 95 Gewichtsprozent an erfindungsgemässer Verbindung bzw. erfindungsgemässen Verbindungen der Formel I als Wirkstoff(en).

Zur Herstellung von pulverförmigen Präparaten kommen inerte pulverförmige Trägerstoffe in Frage, wie z.B. Kaolin, Bentonit, Talkum, Schlämmkreide, Magnesiumcarbonat und Kieselgur. Die aktiven Komponenten werden mit solchen Trägerstoffen vermischt, z.B. durch Zusammenmahlen; oder man imprägniert den inerten Trägerstoff mit einer Lösung der aktiven Komponenten und entfernt dann das Lösungsmittel durch Abdunsten, Erhitzen oder durch Absaugen unter vermindertem Druck. Solche pulverförmigen Präparate können als Stäubemittel mit Hilfe der üblichen Verstäubergeräte auf die zu schützenden Pflanzen aufgebracht werden. Durch Zusatz von Netz- und/oder Dispergiermitteln kann man solche pulverförmigen Präparate mit Wasser leicht benetzbar machen, so dass sie in Form von wässrigen Suspensionen als Spritzmittel anwendbar sind.

Zur Herstellung emulgierbarer Konzentrate können die aktiven Stoffe beispielsweise mit einem Emulgiermittel gemischt oder auch in einem inerten Lösungsmittel gelöst und mit einem Emulgator gemischt werden. Durch Verdünnen solcher

Konzentrate mit Wasser erhält man gebrauchsfertige Emulsionen. Derartige Konzentrate können 5 bis 95 Gewichtsprozent, insbesondere 25 bis 75 Gewichtsprozent, Wirkstoff enthalten.

Die erfindungsgemässen fungiciden Mittel können neben den Wirkstoffen der Formel I auch andere Wirkstoffe enthalten, z.B. anderweitige fungicide Mittel, insecticide und acaricide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums, z.B. durch Reduktion der Anfälligkeit gegen Oidium- und Botrytis-Arten im Rebbau, und können z.B. in Form von pulverförmigen Präparaten oder Spritzbrühen, je nach Einsatzgebiet, vorliegen. Beispiele solcher bekannten Wirkstoffe sind in der folgenden Tabelle zusammengefasst:

| Stoffklasse | Vertreter |
|---|---|
| Dithiocarbamate | Mancozeb (Gemisch von [(1,2-Aethandiylbis(carbamodithioato))(2-)]mangan und [(1,2-Aethandiylbis(carbamodithioato))(2-)]zink |
| Kupferverbindungen | Basisches $CuCl_2$ |
| Bordeaux-Brühe | |
| Schwefel | |
| Phthalimide | Folpet(N-(Trichlormethansulfenyl)-phthalimid) |
| | Captafol (3a,4,7,7a-Tetrahydro-N-(1,1,2,2-tetrachloräthansulfenyl)-phthalimid) |

| | |
|---|---|
| Triphenylzinnverbindungen | Fentinhydroxid (Triphenylzinnhydroxid)<br>Fentinacetat (Triphenylzinnacetat) |
| Benzimidazol-2-carbamidsäure-methylester-Bildner | Benomyl (Methyl-2[1-(butylcarbamoyl)benzimidazolyl]carbamat) |
| | Thiophanate (1,2-Di-(3-äthoxycarbonyl-2-thioureido)benzol) |
| Dicarboximide | Iprodion (3-(3,5-Dichlorphenyl)-1-isopropylcarbamoylhydantoin) |
| | Procymidon (N-(3',5'-Dichlorphenyl)-1,2-dimethylcyclopropan-1,2-dicarboximid) |
| | Vinclozoline (3-(3,5-Dichlorphenyl)-5-methyl-5-vinyl-1,3-oxazolidin-2,4-dion) |
| Nitrile | Chlorothalonil (2,4,5,6-Tetrachlor-1,3-dicyanbenzol) |
| Triazolderivate | Triadimefon (1-(4-Chlorphenoxy)-3,3-dimethyl-1-(1H-1,2,4-triazol-1-yl)-2-butanon) |
| Pyrimidinderivate | Fenarimol (α-(2-Chlorphenyl)-α-(4-chlorphenyl)-5-pyrimidinmethanol) |
| Phosphorsäureester | |
| Carbamate | |
| Pyrethroide | |
| Insektenwuchsregulatoren | |

| | |
|---|---|
| Gibberelline | |
| Düngemittel | auf Stickstoff-, Phosphor-, Kalium-, Calzium- und Spurenelement-Basis. |

Die nachfolgenden Beispiele dienen zur näheren Erläuterung der Erfindung. Die Temperaturangaben beziehen sich auf Celsiusgrade. Sofern nicht anders vermerkt, ist bei der Nennung eines Wirkstoffs der Formel I stets das anfallende racemische Isomerengemisch gemeint.

I. Herstellung der Wirkstoffe:

Beispiel 1

18 g Allyloxyessigsäurechlorid, gelöst in 40 ml Chloroform und 60 ml Toluol, werden mit 40 g wasserfreiem Natriumcarbonat versetzt und 15 Minuten bei Raumtemperatur gerührt. Hierauf werden unter Eiskühlung 20 g 3-[N-(2,6-Dimethylphenyl)]-amino-tetrahydro-2-furanon, gelöst in 60 ml Toluol, zugetropft. Nach 15 Stunden Rühren bei Raumtemperatur wird das Reaktionsgemisch auf Wasser gegossen und mit Essigester extrahiert. Die organische Phase wird nacheinander mit 2N Natronlauge und mit 4N Salzsäure gewaschen, über Natriumsulfat getrocknet, filtriert und eingedampft. Das so erhaltene Rohprodukt wird in wenig Diäthyläther aufgenommen. Das 2-Allyloxy-N-(tetrahydro-2-oxo-3-furyl)-2',6'-acetoxylidid fällt beim Abkühlen kristallin aus dieser Lösung, Smp. 56-58°.

Auf analoge Weise erhält man:

- Aus Propargyloxyessigsäurechlorid und 3-[N-(2,6-Dimethylphenyl)]-amino-tetrahydro-2-furanon das 2-(2-Propinyloxy)-N-(tetrahydro-2-oxo-3-furyl)-2',6'-acetoxylidid, Smp. 160-162°.

- Aus 2-Butenyloxyessigsäurechlorid und 3-[N-(2,6-Dimethylphenyl)]-amino-tetrahydro-2-furanon das 2-(2-Butenyloxy)-N-(tetrahydro-2-oxo-3-furyl)-2',6'-acetoxylidid als gelbliches Oel.

## II. Biologische Beispiele:

### Beispiel 2

### PLASMOPARA VITICOLA

Testmethode:

2 Rebstecklinge der Sorte Riesling x Sylvaner wurden jeweils im 4-5 Blattstadium mit einer wässrigen Dispersion der Testsubstanz (wie allgemein üblich aufbereitet als Spritzpulver) allseitig gründlich besprüht und anschliessend in einer Klimakabine bei 17°, 70-80% relativer Luftfeuchtigkeit und einer Photoperiode von 16 Stunden weiterkultiviert. Nach 7 Tagen erfolgte die Infektion der Versuchspflanzen durch Besprühen der Blattunterseiten mit in destilliertem Wasser suspendierten Zoosporangien (ca. 300'000 Sporangien/ml) von Plasmopara viticola. Danach wurden die Rebpflanzen wie folgt inkubiert: 2 Tage bei 18° und Taupunktbedingungen im Dunkeln und 5 Tage bei 20°, einer relativen Luftfeuchtigkeit von 70-80% und einer Photoperiode von 18 Stunden. Um die Fruktifikation von Plasmopara viticola zu induzieren, wurde am 8. Tag nach der Infektion die relative Luftfeuchtigkeit auf über 90% erhöht.

Die Versuchsauswertung erfolgte jeweils am 9. Tag nach der Infektion durch Ermittlung der durch Plasmopara viticola befallenen Blattfläche in % gegenüber der infizierten, nicht behandelten Kontrolle.

Tabelle I

| Substanz | Konzentration (mg/l Spritzbrühe) | Wirkung (%) |
|---|---|---|
| 2-Allyloxy-N-(tetrahydro-2-oxo-3-furyl)-2',6'-acetoxylidid | 50 | 100 |
| | 16 | 100 |
| | 5 | 92 |

| | | |
|---|---|---|
| 2-(2-Propinyloxy)-N-(tetrahydro-2-oxo-3-furyl)-2',6'-acetoxylidid | 50 | 100 |
| | 16 | 100 |
| | 5 | 100 |

Beispiel 3

PYTHIUM APHANIDERMATUM

Testmethode:

In drei 25 ml-Plastikschalen wurden je zwei mit einer Standard-Düngerlösung getränkte Filterpapierrondellen (Ø 4,5 cm) gegeben. Anschliessend wurden in jede Schale 5 desinfizierte und während 24 Stunden vorgekeimte Gurkensamen (Sorte "Chinesische Schlangen") ausgelegt. Die Kontamination der Samen mit Pythium aphanidermatum erfolgte durch Zugabe von 2 ml einer Mycelsuspension (10 mg Mycelium/ml Wasser) je Schale. Danach wurden die Samen mit einer 1,5 cm dicken Schicht von sterilem Quarzsand bedeckt, mit je 5 ml einer Dispersion der Testsubstanz (als benetzbares Pulver formuliert) behandelt, und anschliessend in einer Klimakabine bei 26°, Taupunktbedingungen und einer Photoperiode von 14 Stunden inkubiert. 6 Tage nach Versuchsbeginn erfolgte die Auswertung durch Auszählen der überlebenden Keimlinge im Vergleich zur infizierten Kontrolle.

Tabelle II

| Substanz | Konzentration (mg/l Sand) | Wirkung (%) |
|---|---|---|
| Erfindungsgemässe Verbindungen: | | |
| 2-Allyloxy-N-(tetrahydro-2-oxo-3-furyl)-2',6'-acetoxylidid | 16 | 100 |
| | 5 | 100 |
| | 1,6 | 100 |
| 2-(2-Propinyloxy)-N-(tetrahydro-2-oxo-3-furyl)-2',6'-acetoxylidid | 16 | 100 |
| | 5 | 100 |
| | 1,6 | 47 |

Stand der Technik (Fenaminosulf):

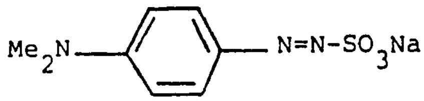

| | | |
|---|---|---|
| | 16 | 100 |
| | 5 | 100 |
| | 1,6 | 0 |

Beispiel 4

TETRANYCHUS OVICID

Testorganismus: Tetranychus urticae, gemeine Spinnmilbe

Testmethode:

Buschbohnen-Blattrondellen (Ø 25 mm) werden mit adulten Weibchen infiziert. 1 Tag später werden die Weibchen entfernt und die Blattrondellen mit ca. 30-50 Eiern werden mit acetonischer Wirkstofflösung besprüht. Die behandelten Blattrondellen werden auf feuchten Schaumstoff gelegt und bei 25° und 60% relativer Feuchtigkeit inkubiert. Unbehandelte und mit Aceton behandelte Rondellen dienen als Kontrollparzellen. Testdauer: 6 Tage. Die Resultate werden ausgedrückt als prozentuale Reduktion der Ueberlebensrate im Vergleich zu den Kontrollparzellen. Die Resultate sind in nachstehender Tabelle III zusammengefasst:

Tabelle III

| Dosierung ($10^{-x}$ g AS/$cm^2$): | 5 | 6 |
|---|---|---|
| | % Wirkung | |
| Stand der Technik (Metalaxyl): N-Methoxyacetyl-N-(2,6-xylyl)-alaninmethylester | 0 | 0 |
| Erfindungsgemässe Verbindungen: | | |
| 2-Allyloxy-N-(tetrahydro-2-oxo-3-furyl)-2',6'-acetoxylidid | 100 | 8 |
| 2-(2-Propinyloxy)-N-(tetrahydro-2-oxo-3-furyl-2',6'-acetoxylidid | 100 | 3 |

(Unbehandelte Kontrollen: 3% Mortalität)
AS = Wirkstoff

III. Formulierungsbeispiele:

Beispiel 5

| | |
|---|---|
| Wirkstoff (flüssig) der Formel I | 750 Gew.-Teile |
| Ricinusöl-Aethylenoxid-Addukt | 100 " |
| Ca-Dodecylbenzolsulfonat | 25 " |
| Aromatisches Lösungsmittel | |
| (Gemisch von $C_{10}$-Alkylbenzolen) zu 1000 Vol.-Teilen | |

Die Komponenten werden im Lösungsmittel gelöst. Das entstandene emulgierbare Konzentrat wird zur Herstellung der gebrauchsfertigen Spritzbrühe in Wasser gegeben, wobei eine für Stunden stabile Emulsion (Oel/Wasser) entsteht.

Beispiel 6

| | |
|---|---|
| Wirkstoff (fest) der Formel I | 80 Gew.-Teile |
| Kieselsäure, hydratisiert | 10 " |
| Na-Laurylsulfat | 1 " |
| Na-Lignosulfonat | 2 " |
| Kaolin (Kaolinit) | 7 " |

Die Komponenten werden in einer Mischmaschine gemischt, diese Mischung in einer Luftstrahlmühle gemahlen und hernach wieder gemischt. Das entstandene Spritzpulver, in welchem der Wirkstoff in einer Partikelgrösse von unter 10 Mikron vorliegt, lässt sich mit Wasser gut benetzen und ergibt eine gut schwebefähige Dispersion zur Behandlung von Pflanzen.

Patentansprüche

1. Verbindungen der allgemeinen Formel

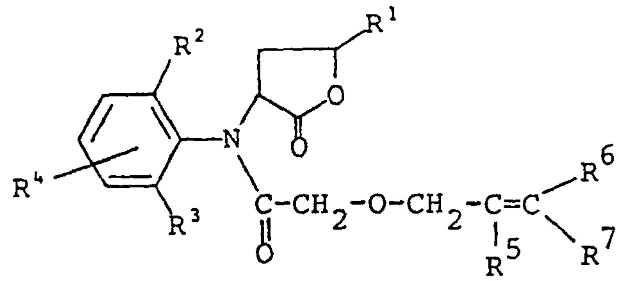

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt.

2. Verbindungen nach Anspruch 1, worin $R^1$ Wasserstoff bedeutet.

3. Verbindungen nach Anspruch 1 oder 2, worin $R^4$ Wasserstoff bedeutet.

4. Verbindungen nach Anspruch 3, worin die substituierte Phenylgruppe 2,6-Dichlorphenyl, 2-Chlor-6-methylphenyl oder 2,6-Dimethylphenyl ist.

5. 2-Allyloxy-N-(tetrahydro-2-oxo-3-furyl)-2',6'-acetoxylidid.

6. 2-(2-Propinyloxy)-N-(tetrahydro-2-oxo-3-furyl)-2',6'-acetoxylidid.

7. 2-(2-Butenyloxy)-N-(tetrahydro-2-oxo-3-furyl)-2',6'-acetoxylidid.

8. Verbindungen nach einem der Ansprüche 1 bis 7 als fungicide Wirkstoffe.

9. Fungicides Mittel, dadurch gekennzeichnet, dass es eine wirksame Menge von mindestens einer Verbindung der allgemeinen Formel

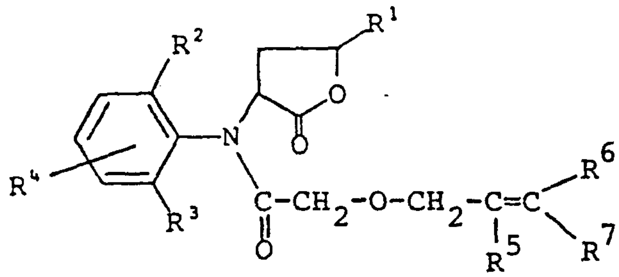

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt, sowie inertes Trägermaterial enthält.

10. Fungicides Mittel nach Anspruch 9, dadurch gekennzeichnet, dass es eine wirksame Menge von 2-Allyloxy-N-(tetrahydro-2-oxo-3-furyl)-2',6'-acetoxylidid sowie inertes Trägermaterial enthält.

11. Fungicides Mittel nach Anspruch 9, dadurch gekennzeichnet, dass es eine wirksame Menge von 2-(2-Propinyloxy)-N-(tetrahydro-2-oxo-3-furyl)-2',6'-acetoxylidid sowie inertes Trägermaterial enthält.

12. Verfahren zur Herstellung von Verbindungen der allgemeinen Formel

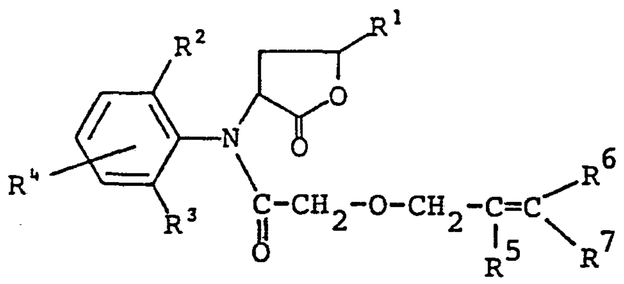

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt,

dadurch gekennzeichnet, dass man eine Verbindung der Formel

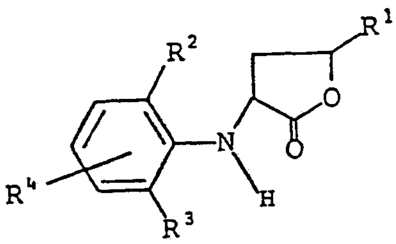

II

worin $R^1$, $R^2$, $R^3$ und $R^4$ die oben angegebenen Bedeutungen besitzen,

mit einer Carbonsäure der Formel

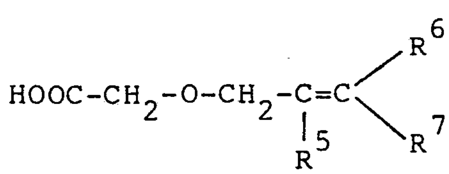

III

worin $R^5$, $R^6$ und $R^7$ die oben angegebenen Bedeutungen besitzen,

oder einem reaktionsfähigen Säurehalogenid, Säureanhydrid, Ester oder Amid davon umsetzt.

13. Verfahren nach Anspruch 12 zur Herstellung von 2-Allyloxy-N-(tetrahydro-2-oxo-3-furyl)-2',6'-acetoxylidid, dadurch gekennzeichnet, dass man 3-[N-(2,6-Dimethylphenyl)]-amino-tetrahydro-2-furanon mit Allyloxyessigsäurechlorid umsetzt.

14. Verfahren nach Anspruch 12 zur Herstellung von 2-(2-Propinyloxy)-N-(tetrahydro-2-oxo-3-furyl)-2',6'-acetoxylidid, dadurch gekennzeichnet, dass man Propargyloxyessigsäurechlorid mit 3-[N-(2,6-Dimethylphenyl)]-amino-tetrahydro-2-furanon umsetzt.

15. Verfahren zur Bekämpfung von Fungi in der Landwirtschaft und im Gartenbau, dadurch gekennzeichnet, dass man das zu schützende Gut mit einer wirksamen Menge einer der in den Ansprüchen 1 bis 7 genannten Verbindungen bzw. eines der in den Ansprüchen 9 bis 11 genannten Mittel behandelt.

16. Verwendung einer der in den Ansprüchen 1 bis 7 genannten Verbindungen bzw. eines der in den Ansprüche 9 bis 11 genannten Mittel zur Bekämpfung von Fungi in der Landwirtschaft und im Gartenbau.

***

Patentansprüche
"für OESTERREICH"

1. Fungicides Mittel, dadurch gekennzeichnet, dass es eine wirksame Menge von mindestens einer Verbindung der allgemeinen Formel

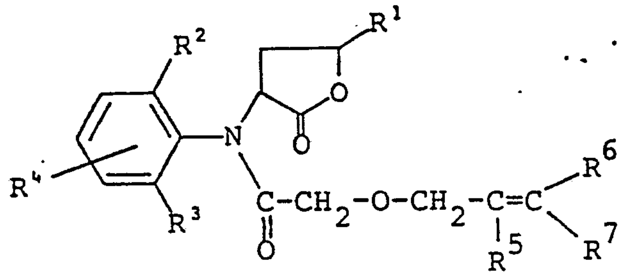

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt, sowie inertes Trägermaterial enthält.

2. Fungicides Mittel nach Anspruch 1, worin $R^1$ Wasserstoff bedeutet.

3. Fungicides Mittel nach Anpruch 1 oder 2, worin $R^4$ Wasserstoff bedeutet.

4. Fungicides Mittel nach Anspruch 3, worin die substituierte Phenylgruppe 2,6-Dichlorphenyl, 2-Chlor-6-methylphenyl oder 2,6-Dimethylphenyl ist.

5. Fungicides Mittel nach Anspruch 1, dadurch gekennzeichnet, dass es eine wirksame Menge von 2-Allyloxy-N-(tetrahydro-2-oxo-3-furyl)-2',6'-acetoxylidid sowie inertes Trägermaterial enthält.

6. Fungicides Mittel nach Anspruch 1, dadurch gekennzeichnet, dass es eine wirksame Menge von 2-(2-Propinyloxy)-N-(tetrahydro-2-oxo-3-furyl)-2',6'-acetoxylidid sowie inertes Trägermaterial enthält.

7. Fungicides Mittel nach Anspruch 1, dadurch gekennzeichnet, dass es eine wirksame Menge von 2-(2-Butenyloxy)-N-(tetrahydro-2-oxo-3-furyl)-2',6'-acetoxylidid sowie inertes Trägermaterial enthält.

8. Verfahren zur Herstellung von Verbindungen der allgemeinen Formel

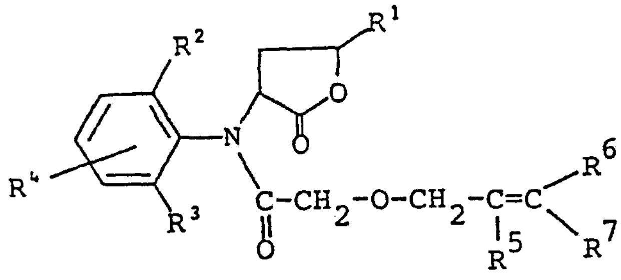

I

worin $R^1$ Wasserstoff oder Methyl, $R^2$ und $R^3$ Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen und $R^5$, $R^6$ und $R^7$ Wasserstoff oder Methyl bedeuten, wobei $R^5$ zusammen mit $R^6$ auch eine zusätzliche C-C-Bindung darstellen kann und wobei die Gesamtzahl der Kohlenstoffatome in den Substituenten $R^2$, $R^3$ und $R^4$ 6 nicht übersteigt,

dadurch gekennzeichnet, dass man eine Verbindung der Formel

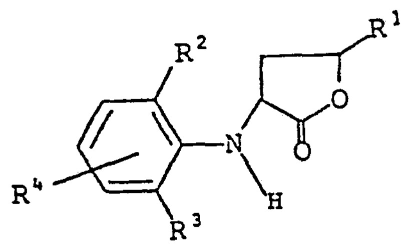

II

worin $R^1$, $R^2$, $R^3$ und $R^4$ die oben angegebenen Bedeutungen besitzen,

mit einer Carbonsäure der Formel

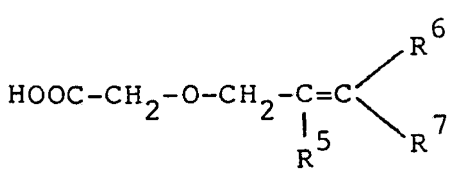

III

worin $R^5$, $R^6$ und $R^7$ die oben angegebenen Bedeutungen besitzen,

oder einem reaktionsfähigen Säurehalogenid, Säureanhydrid, Ester oder Amid davon umsetzt.

9. Verfahren nach Anspruch 8 zur Herstellung von 2-Allyloxy-N-(tetrahydro-2-oxo-3-furyl)-2',6'-acetoxylidid, dadurch gekennzeichnet, dass man 3-[N-(2,6-Dimethylphenyl)]-amino-tetrahydro-2-furanon mit Allyloxyessigsäurechlorid umsetzt.

10. Verfahren nach Anspruch 8 zur Herstellung von 2-(2-Propinyloxy)-N-(tetrahydro-2-oxo-3-furyl)-2',6'-acetoxylidid, dadurch gekennzeichnet, dass man Propargyloxyessigsäurechlorid mit 3-[N-(2,6-Dimethylphenyl)]-amino-tetrahydro-2-furanon umsetzt.

11. Verfahren zur Herstellung eines fungiciden Mittels, dadurch gekennzeichnet, dass man mindestens eine der in den Ansprüchen 1 bis 6 genannten Verbindungen mit inertem Trägermaterial vermischt.

12. Verfahren zur Herstellung eines fungiciden Mittels, dadurch gekennzeichnet, dass man 2-(2-Butenyloxy)-N-(tetrahydro-2-oxo-3-furyl)-2',6'-acetoxylidid mit inertem Trägermaterial vermischt.

13. Verfahren zur Bekämpfung von Fungi in der Landwirtschaft und im Gartenbau, dadurch gekennzeichnet, dass man das zu schützende Gut mit einer wirksamen Menge eines der in den Ansprüchen 1 bis 6 genannten Mittel behandelt.

14. Verfahren zur Bekämpfung von Fungi in der Landwirtschaft und im Gartenbau, dadurch gekennzeichnet, dass man das zu schützende Gut mit einer wirksamen Menge eines in Anspruch 7 genannten Mittels behandelt.

15. Verwendung einer bzw. eines der in den Ansprüchen 1 bis 6 genannten Verbindungen bzw. Mittel zur Bekämpfung von Fungi in der Landwirtschaft und im Gartenbau.

16. Verwendung der bzw. des in Anspruch 7 genannten Verbindung bzw. Mittels zur Bekämpfung von Fungi in der Landwirtschaft und im Gartenbau.

***

0033516

Europäisches Patentamt

**EUROPÄISCHER RECHERCHENBERICHT**

Nummer der Anmeldung
EP 81 10 0630

| EINSCHLÄGIGE DOKUMENTE | | | KLASSIFIKATION DER ANMELDUNG (Int Cl 3) |
|---|---|---|---|
| Kategorie | Kennzeichnung des Dokuments mit Angabe soweit erforderlich der maßgeblichen Teile | betrifft Anspruch | |
| | DE - A - 2 804 299 (CIBA-GEIGY)<br>* Ansprüche *<br>-- | 1-4,8, 9,12, 15,16 | C 07 D 307/32<br>A 01 N 43/08 |
| | DE - A - 2 845 454 (CHEVRON)<br>* Ansprüche *<br>-- | 1,8,9, 15,16 | |
| | DE - A - 2 847 075 (CHEVRON)<br>* Ansprüche *<br>-- | 1,12 | RECHERCHIERTE SACHGEBIETE (Int Cl 3) |
| P | DE - A - 2 841 824 (SCHERING)<br>* Ansprüche, insbesondere Anspruch 2 *<br>-- | 1-4,8, 9,12, 15,16 | C 07 D 307/32<br>A 01 N 43/08 |
| P | EP - A - 0 018 510 (CIBA-GEIGY)<br>* Ansprüche *<br>---- | 1,8,9, 12,15, 16 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 15-04-1981 | CHOULY |